# EUROPÄISCHE PATENTANMELDUNG

(11) **EP 4 467 034 A1**
(43) Veröffentlichungstag der Anmeldung: **27.11.2024**
(21) Anmeldenummer: 23175305.4
(22) Anmeldetag: 25.05.2023
(51) Int. Cl.: A43D 1/02

(54) **VORRICHTUNG ZUR ELEKTRONISCHEN ERFASSUNG EINES DYNAMISCHEN UND/ODER STATISCHEN FUSSABDRUCKS**

(71) Anmelder: medi GmbH & Co. KG, 95448 Bayreuth (DE)
(72) Erfinder: HERTZ, Thomas, 95448 Bayreuth (DE); RIEDEL, Robert, 95448 Bayreuth (DE)
(74) Vertreter: Appelt, Christian W.

(57) **Zusammenfassung**

Die Erfindung betrifft eine Vorrichtung zur elektronischen Erfassung eines dynamischen und/oder statischen Fußabdrucks mit einer flächigen Drucherfassungseinrichtung, die eine Vielzahl von Drucksensoren beinhaltet. Die Vorrichtung umfasst ferner eine Verarbeitungseinheit, die eingerichtet ist, die Messung der auf die Fläche der Druckerfassungseinrichtung wirkenden Kräfte des Fußes mittels des Erfassungssignals zu veranlassen und basierend auf den Ergebnissen ein Abbild der Kräfteverteilung des Fußes auf der Druckerfassungseinrichtung zu erstellen.

## Beschreibung

### Technisches Gebiet

Die Erfindung betrifft die elektronische Erfassung von Fußabdrücken, insbesondere dynamischen Fußabdrücken und/oder statischen Fußabdrücke, die beispielsweise im Rahmen einer Ganganalyse erfasst werden können. Die so gewonnenen Daten können beispielsweise für die Anfertigung von individuellen (orthopädischen) Schuheinlagen verwendet werden.

### Technischer Hintergrund

Die Pedographie betrifft die analoge oder digitale Darstellung der Druckbelastung eines Fußes während des Stands oder beim Gehen in Form eines sogenannten Programms.

Dazu kommen herkömmlicherweise unterschiedliche Methoden zum Einsatz, die die Druckbelastung eines Fußes zu erfassen. Die wohl verbreitetste Möglichkeit der Darstellung der Druckbelastung ist der Blauabdruck. Dieser ist eine zweidimensionale Methode zur Vermessung des Fußes und funktioniert ähnlich wie ein Stempelkissen. Ein Blauabdruck wird herkömmlicherweise mittels einer Blauabdruckplatte erstellt. Der Proband steht auf der Abdruckplatte, die auf der Gegenseite eine Blaupause aufweist. Durch das Körpergewicht wird der Fußabdruck auf ein innenliegendes Papier abgebildet. Typischerweise wird ferner mit einem Kunststoffstab der Umriss des Fußes nachgezeichnet. Der Blauabdruck stellt eine Momentaufnahme der Druckverteilung des Fußes dar, die nicht reproduziert werden kann. Je nachdem, wie viel Tusche man aufträgt, ist das Farbbild heller oder dunkler. Dies kann die Interpretation des Farbbildes subjektiv beeinflussen. Es ist auch nicht nachvollziehbar, ob der Proband gerade oder schief auf der Platte steht. Auch weist bei einer Blaupause die zugehörige Abdruckplatte auf der Innenseite Erhebungen auf, sodass bei der Druckausübung punktförmige Abbildungen in einem sehr groben Raster erzeugt werden.

Es existieren im Stand der Technik auch digitale Erfassungssysteme, die den Fußabdruck eines Probanden erfassen können. Bei einem 2D oder 3D Computerscan wird der Fuß auf einer Glasplatte von einem Streifenscanner abgetastet. Beispielsweise offenbart die DE 20 2007 011 702 U1 ein optisches System, dass in der Lage ist einen dynamischen Fußabdruck mit optischen Mitteln zu erfassen. Alternativ ist auch eine mechanische Abtastung, beispielsweise durch Stifte, die sich gegen die Fußsohle bewegen und der Erfassung ihres Anschlagspukts an der Fußsohle, möglich.

Eine weitere Möglichkeit der Fußdruckmessung (Pedobarografie) ist die Verwendung dünner elektronischer Messsohlen. Eine solche "Messsohle" ist beispielweise aus der WO 2014/169244 A2 bekannt. Solche Schuhsohlen können Impulse der wirkenden Kräfte beim Gehen (dynamisch) oder Stehen (statisch) aufzeichnen.

Auch kommen teilweise elektronische Druckmessplatten zum Einsatz, die die Druckverteilung des Fußes durch das Gehen oder Stehen auf einer druckempfindlichen Platte oder einfach die Schuhgröße des Probanden elektronisch erfassen. In der CA 2003269 A1 wird beispielsweise ein System beschrieben, mit dem der Druck, der von den Füßen einer Person ausgeübt wird, erfasst und bestimmt werden kann, um so die Fußabdrücke zu ermitteln. Das System verwendet eine Vielzahl von Sensoren, die gleichmäßig auf dem Trittbrett und unter einer Schicht aus flexiblem Material, wie z. B. Gummi, verteilt sind. Die Sensoren sind mit einem Multiplexer, einem Analog/DigitalWandler und einem programmierten Rechner verbunden, der die Druckdaten in sichtbare und/oder lesbare Werte umsetzt.

Bei diesen elektronischen Erfassungssystemen, insbesondere den elektronischen Druckmessplatten, hat sich als nachteilig herausgestellt, dass die Auflösung der Drucksensoren ungenügend ist, um ein ausreichend genaues Abbild der Druckverteilung des Fußabdrucks zu ermitteln. So kommen in der CA 2003269 A1 lediglich 4928 Sensoren zum Einsatz. Auch ist es bei solchen elektronischen Systemen nicht möglich, den Fußumriss zu erfassen.

### Zusammenfassung der Erfindung

Ausgehend von diesem Hintergrund, stellt sich die vorliegende Erfindung die Aufgabe, ein System (insb. eine Vorrichtung) zur Erfassung von Fußabdrücken zur Verfügung zu stellen, das gegenüber den im Stand der Technik bekannten Lösungen verbessert ist. Eine weitere Aufgabe der Erfindung besteht darin, ein solches System zur Verfügung zu stellen, welches einfach zu bedienen ist. Vorteilhafterweise soll ein solches System auch portabel sein, damit es der Orthopädietechniker einfach transportieren und beim Kunden einsetzen kann.

Ein Aspekt der vorliegenden Erfindung besteht darin, die Auflösung der Abbildung der Druckverteilung eines Fußes durch das Vorsehen einer besonders großen Anzahl von Drucksensoren pro Flächeneinheit in der elektronischen Druckplatte signifikant zu erhöhen, sodass sich anhand des digitalen Abbilds der Druckverteilung des Fußes auf der Druckplatte die Druckverteilung so exakt erfasst werden kann, dass basierend auf den digital erfassten Daten der Druckverteilung des Fußes individuell angefertigte (orthopädische) Schuheinlagen mit großer Präzision angefertigt werden können.

Ein weiterer Aspekt der Erfindung betrifft die Möglichkeit, mittels der elektronischen Druckplatte nicht nur den statischen und/oder dynamischen Fußabdruck des Probanden zu erfassen, sondern gleichzeitig auch den Fußumriss des Probanden erfassen zu können.

Eine Ausführungsform der Erfindung betrifft eine Vorrichtung, die eine flächige Druckerfassungseinrichtung aufweist. Die Druckerfassungseinrichtung verfügt über eine Vielzahl von Drucksensoren in einer vorgegebenen zweidimensionalen Anordnung, bspw. in einer x-y Ebene. Diese vorgegebene zweidimensionale Anordnung der Drucksensoren weist eine Dichte von mindestens 20 Drucksensoren pro 1 cm², bevorzugt 25 Drucksensoren pro 1 cm², bevorzugt von mindestens 35 Drucksensoren pro 1 cm², und weiter bevorzugt mindestens 40 Drucksensoren pro 1 cm² auf. Bei einer üblichen Fläche der Druckerfassungseinrichtung von ca. 40 cm x 20 cm können somit mindestens 16.000 Drucksensoren, bevorzugt ca. 30.000 Drucksensoren zur Erfassung der Druckverteilung verwendet werden und so ein besonders genaues Abbild der Druckverteilung des Fußes auf die Druckerfassungseinrichtung erfasst werden.

Es soll an dieser Stelle angemerkt werden, dass die Vielzahl der Drucksensoren zwar in einer zweidimensionalen Ebene an unterschiedlichen Punkten oder Positionen angeordnet sind, um die zweidimensionale Ebene über einen vorgegebenen Bereich und in einer vorgegebenen Dichte relativ zu der zweidimensionalen Ebene abzudecken, es aber nicht erforderlich ist, dass sämtliche Drucksensoren zwangsweise auf exakt der gleichen Höhe relativ oder normal zu der zweidimensionalen Ebene oder der x-y Ebene angeordnet sind. Vielmehr mag es sinnvoll sein, die Drucksensoren in leicht unterschiedlichen Höhen, also in einer Richtung relativ zu der x-y Ebene, anzuordnen, um eine möglichst hohe Dichte, in der x-y Ebene, zu erzeugen.

Die Druckerfassungseinrichtung ist eingerichtet, die auf eine Fläche der Druckerfassungseinrichtung wirkenden Kräfte eines Fußes mittels der Drucksensoren zu einem Erfassungszeitpunkt zu messen. Die Druckerfassungseinrichtung kann dabei auf ein Erfassungssignal reagieren. Dieses Erfassungssignal bewirkt, dass die Druckerfassungseinrichtung die wirkenden Kräfte des Fußes mittels der Drucksensoren zu dem Erfassungszeitpunkt ermittelt. Ferner kann das Erfassungssignal (optional) den Betriebsmodus der Druckerfassungseinrichtung vorgeben. So kann beispielsweise die Druckerfassungseinrichtung veranlasst werden, einen statischen Fußabdruck oder einen dynamischen Fußabdruck zu erfassen.

Die Vorrichtung umfasst ferner eine Verarbeitungseinheit. Die Verarbeitungseinheit ist eingerichtet, basierend auf den mittels der einzelnen Drucksensoren zum Erfassungszeitpunkt erfassten Kräften ein Abbild der Kräfteverteilung des Fußes auf der Druckerfassungseinrichtung zu erstellen. Dieses Abbild kann bevorzugt in einer Darstellung wiedergegeben werden, die, über die zweidimensionale Verteilung der Drucksensoren, jedem einzelnen Sensor einen entsprechenden Druckwert zuordnet. Bevorzugt wird jedem Sensor, der in einer x-y Ebene angeordnet ist, ein entsprechender Druckwert zugeordnet. Dies kann als Tabelle dargestellt werden, allerdings ist es auch möglich, eine dreidimensionale Darstellung dieses Abbilds zu erzeugen, indem beispielsweise jedem Drucksensor ein Druckwert zugeordnet wird, der in Form einer Säule oder eines Balken wiedergegeben wird. Ferner kann die Verarbeitungseinheit auch optional eingerichtet sein, die Messung der auf die Fläche der Druckerfassungseinrichtung wirkenden Kräfte des Fußes mittels des Erfassungssignal zu veranlassen.

In einer weiteren Ausführungsform der Erfindung sind die Drucksensoren in der Druckerfassungseinrichtung in einer im Wesentlichen rechteckigen Fläche angeordnet. Die rechteckige Fläche weist dabei beispielsweise Seitenverhältnis im Bereich 1:1,5 und 1:2,5, bevorzugt 1:2 auf. Grundsätzlich ist es aber auch denkbar, dass die Drucksensoren nicht in einer rechteckigen Fläche angeordnet sind. Beispielsweise kann die flächige Anordnung der Drucksensoren auch der Form des Fußes nachempfunden sein. In einem Ausführungsbeispiel der Erfindung sind die Drucksensoren in der Druckerfassungseinrichtung in einer im Wesentlichen rechteckigen Fläche von ca. 40cm x ca. 20 cm angeordnet.

Bei einer besonders bevorzugten Ausführungsform können die Drucksensoren und/oder die Druckerfassungseinrichtung in einem mobilen Gerät angeordnet sein, das mit einem Akku/einer Batterie ausgestattet ist und/oder das mittels eines Netzteils an eine Stromversorgung anschließbar ist. Bei der besonders bevorzugten Ausführungsform ist dieses mobile Gerät mit einer Kommunikationsvorrichtung ausgestattet, die eine Verbindung mit einem Computer und/oder einem Netzwerk oder einer anderen mobilen Vorrichtung ermöglicht. Insbesondere kann das Mobilgerät beispielsweise mit einer Bluetooth Funktionalität oder einer WLAN Funktionalität ausgestattet sein und/oder entsprechende Bluetooth und/oder WLAN Sende- und/oder Empfangsbauteile umfassen und/oder über ein Mobilfunknetz mit anderen Geräten verbindbar sein.

Ferner kann die Druckerfassungseinrichtung gemäß einer weiteren Ausführungsform der Erfindung eingerichtet ist, die auf die Fläche der Druckerfassungseinrichtung wirkenden Kräfte des Fußes mittels der Drucksensoren kontinuierlich in Intervallen zu unterschiedlichen Erfassungszeitpunkten zu ermitteln. Die Verarbeitungseinheit ist ferner eingerichtet ist, basierend auf den mittels der einzelnen Drucksensoren zu den unterschiedlichen Erfassungszeitpunkten erfassten Kräften eine zeitliche Sequenz mehrerer Abbilder der Kräfteverteilung des Fußes auf der Druckerfassungseinrichtung zu erstellen. Beispielsweise kann die Druckerfassungseinrichtung die auf die einzelnen Drucksensoren wirkenden Kräfte in Intervallen von 5 ms oder weniger, bevorzugt 1 ms erfassen und in entsprechenden Intervallen periodisch an die Verarbeitungseinrichtung ausgeben.

Die Verarbeitungseinheit kann optional ferner eingerichtet sein, aus den (unterschiedlichen) Abbildern der Kräfteverteilung des Fußes auf der Druckerfassungseinrichtung durch Mittelwertbildung der jeweiligen zu den unterschiedlichen Messzeitpunkten auf die jeweiligen Drucksensoren wirkenden Kräfte einen dynamischen Fußabdruck zu erzeugen.

Gemäß einer weiteren Ausführungsform der Erfindung kann die Druckerfassungseinrichtung eingerichtet sein, die auf die Fläche der Druckerfassungseinrichtung wirkenden Kräfte des Fußes mittels der Drucksensoren kontinuierlich in Intervallen zu unterschiedlichen Erfassungszeitpunkten zu ermitteln. Dies kann in Reaktion auf ein zweites Erfassungssignal geschehen. Die Verarbeitungseinheit ist hierbei weiter eingerichtet, für jeden der Drucksensoren einen Mittelwert der durch den jeweiligen Drucksensor zu den unterschiedlichen Erfassungszeitpunkten erfassten Kräfte zu ermitteln und basierend auf den Mittelwerten ein dynamisches Abbild der Kräfteverteilung des Fußes auf der Druckerfassungseinrichtung zu erstellen. Auch in diesem Ausführungsbeispiel können die Intervalle bevorzugt 5 ms oder weniger betragen, besonders bevorzugt 1 ms.

In einer weiteren Ausführungsform der Erfindung ist die Druckerfassungseinrichtung eingerichtet, die auf jeden Drucksensor in einem Messintervall maximal wirkende Druckkraft zu ermitteln, die von dem Fuß auf die Fläche der Druckerfassungseinrichtung auf den jeweiligen Drucksensor ausgeübt wird. Dies kann in Reaktion auf ein drittes Erfassungssignal geschehen. Die Verarbeitungseinheit eingerichtet ist, basierend auf den maximalen Druckwerten ein statisches Abbild der Kräfteverteilung des Fußes auf der Druckerfassungseinrichtung im Messintervall zu erstellen. In diesem Modus ist das Messintervall deutlich größer als das Intervall zur Ermittlung von dynamischen Abdrücken. Das Messintervall kann dabei zwischen 1 Sekunde und 240 Sekunden (oder auch länger) betragen. Bevorzugt liegt ein Messintervall im Bereich von 1 Sekunde bis 10 Sekunden.

Gemäß einer besonderen Ausführungsform umfasst die Vorrichtung insbesondere ein Eingabemittel, bevorzugt einen elektronischen, aktiven oder passiven, Stift, der so ausgebildet ist, dass der Nutzer mittels dieses Eingabemittels einen Fuß umfahren kann. Durch dieses Eingabemittel, insbesondere durch den Stift, ist es besonders einfach möglich, den Umriss des Fußes durch die Vorrichtung zu erfassen. Bevorzugt wird ein Druck, der durch das Eingabemittel, insbesondere den Stift, auf die Druckerfassungseinrichtung ausgeübt wird, von den entsprechenden Drucksensoren umfasst. Alternativ ist es auch möglich, dass das Eingabemittel kapazitiv mit der Vorrichtung verbunden ist, sodass zwischen Eingabemittel, insbesondere Stift, und Druckerfassungseinrichtung eine kapazitive Kopplung stattfindet, mittels der die Eingabe und insbesondere der Fußumriss ermittelt wird.

Besonders bevorzugt ist dabei die Vorrichtung so ausgebildet, dass auf der Basis der Messergebnisse, insbesondere auf der Basis der von den Drucksensoren ermittelten Druckwerte und/oder auf der Basis der kapazitiven Kopplung zwischen Eingabemittel, insbesondere Stift, und Druckerfassungseinrichtung, eine Fußgröße ermittelt wird. Zur Ermittlung der Fußgröße kann bei einer besonderen Ausführungsform insbesondere auf in der Vorrichtung selbst gespeicherte Daten oder eine Datenbank zurückgegriffen werden, wobei die von der Vorrichtung ermittelten Messwerte mit den gespeicherten Werten in Beziehung gesetzt werden. Insbesondere ist es beispielsweise möglich, den maximalen Abstand zwischen zwei Drucksensoren zu bestimmen, die jeweils mit einem Druck beaufschlagt sind bzw. mit einem Druck über einem vorbestimmten Schwellwert beaufschlagt sind, sodass aus diesem maximalen Abstand zwischen den zwei Drucksensoren eine Fußgröße ermittelt werden kann.

Bevorzugt ist die Vorrichtung so ausgebildet, dass nach der Ermittlung eines Umrisse des Fußes, insbesondere mittels des Eingabemittels, bevorzugt des Stiftes, ein erweiterter Umriss berechnet und/oder ermittelt wird, beispielsweise durch automatische Vergrößerung des Umrisse, beispielsweise um 0,5 cm bis 1,5 cm oder um einen anderen festgelegten Wert, der beispielsweise auch in Abhängigkeit von den absolut gemessenen Druckwerten der einzelnen Drucksensoren und/oder einem entsprechenden Mittelwert und damit vom Gewicht des Nutzers abhängt, sodass eine bevorzugte Sohlengröße bestimmt werden kann.

Gemäß einer weiteren Ausführungsform kann die Druckerfassungseinrichtung eingerichtet sein, die auf jeden Drucksensor in einem zweiten Messintervall maximal wirkende Druckkraft zu ermitteln, die von dem Fuß bzw. einem Eingabemittel, mit dem der Fuß umfahren wird, auf die Fläche der Druckerfassungseinrichtung auf den jeweiligen Drucksensor ausgeübt wird. Dies kann beispielsweise in Reaktion auf ein viertes Erfassungssignal erfolgen. Ohne dass die Erfindung darauf beschränkt ist, kommen als Eingabemittel beispielsweise -je nach Ausgestaltung der Drucksensoren - ein elektronischer aktiver oder passiver Stift, ein Kunststoffstab, ein Finger, oder dergleichen infrage. Die Verarbeitungseinheit ist ferner eingerichtet, basierend auf den maximalen Druckwerten ein zweites statisches Abbild der Kräfteverteilung des Fußes und/oder des Eingabemittel auf der Druckerfassungseinrichtung im Messintervall zu erstellen.

Optional kann die Verarbeitungseinheit auch eingerichtet sein, den mit dem Eingabemittel erzeugten Teil der im Erfassungsintervall auf die Fläche wirkenden Kräfte durch Bildung eines Differenzbildes aus dem zweiten Abbild und dem ersten Abbild der Kräfteverteilung des Fußes auf der Druckerfassungseinrichtung zu ermitteln. Aus diesem Differenzbild lässt sich so der Umriss des Fußes ermitteln.

Gemäß einem weiteren Ausführungsbeispiel der Erfindung kann die Verarbeitungseinheit ferner eingerichtet sein, Druckdifferenzen im Differenzbild, die kleiner einem vorgegeben oder konfigurierbaren Schwellenwert sind, bei der Ermittlung des Umrisses zu ignorieren.

Beispielsweise lässt sich so die Ermittlung des Fußumrisses verbessern, wenn der Proband sich zwischen den beiden Aufnahmen der Druckverteilung bewegt. Alternativ kann die Verarbeitungseinheit auch eingerichtet sein, lediglich Druckdifferenzen im Differenzbild, die größer als ein vorgegebener Schwellenwert sind zu berücksichtigen.

Ferner kann die Verarbeitungseinheit auch eingerichtet sein, basierend auf dem ermittelten Umriss des Fußes die Fußmittelachse zu bestimmen. Dabei kann die Verarbeitungseinheit optional eingerichtet sein, sowohl die Fußmittelachse für einen Umriss des Fußes, der auf einem Mittelwert-basierten, Abbild der Kräfteverteilung des Fußes ermittelt wurde, als auch die Fußmittelachse für einen Umriss des Fußes, der auf einem Einzelmessung-basierten Abbild der Kräfteverteilung des Fußes ermittelt wurde, zu errechnen.

Weitere Ausführungsformen der Erfindung betreffen die Darstellung der unterschiedlichen Abbildungen (statische Fußabdruck, dynamischen Fußabdruck, Fußumriss). Diese können beispielsweise mittels einer Anzeigevorrichtung dargestellt werden und/oder mit einem (farbigen) Drucker ausgegeben werden. Die unterschiedlichen erfassten Abbildungen können jedoch auch rein digital gespeichert werden, was die Datenübermittlung ermöglicht. So könnten beispielsweise in einer Orthopädiewerkstatt die erfassten Abbilder automatisch ausgewertet werden und/oder direkt in eine Fertigungsvorlage für eine (orthopädische) Schuheinlage umgesetzt werden.

In einer weiteren Ausführungsform der Erfindung umfasst die Vorrichtung eine Anzeigeeinheit, die eingerichtet ist, den Umriss des Fußes und das Abbild der Kräfteverteilung des Fußes anzuzeigen.

Die Verarbeitungseinheit kann dazu eingerichtet ist, die unterschiedlichen Kräfte des Abbilds der Kräfteverteilung des Fußes entsprechend der jeweiligen Position der Drucksensoren in der vorgegebenen zweidimensionalen Anordnung in unterschiedlichen Farben und/oder Farbabstufungen darzustellen. Ferner oder alternativ dazu kann die Verarbeitungseinheit eingerichtet sein, die den Umriss des Fußes als Umrisslinie in der Darstellung des zweidimensionalen Abbilds der Kräfteverteilung des Fußes zu kennzeichnen. Die Verarbeitungseinheit ist optional auch eingerichtet, die Fußmittelachse in der Darstellung des zweidimensionalen Abbilds der Kräfteverteilung des Fußes zu kennzeichnen.

Eine weitere Ausführungsform der Erfindung betrifft die Implementierung der bereits exemplarisch dargelegten unterschiedlichen Erfassungsmethoden mittels eines Computer-gestützten Verfahrens. Eine Ausführungsform der Erfindung ist daher ein computer-gestütztes Verfahren zur Ermittlung eines Fußabdrucks, das von der Verarbeitungseinheit durchgeführt werden kann. Das Verfahren umfasst das Empfangen von Messwerten von einer Druckerfassungseinrichtung, wobei die Messwerte die auf die jeweiligen Drucksensoren der Druckerfassungseinrichtung wirkenden Kräfte eines Fußes zu einem Erfassungszeitpunkt repräsentieren. Ferner umfasst das Verfahren ein Erstellen eines zweidimensionalen Abbilds der Kräfteverteilung des Fußes auf der Druckerfassungseinrichtung basierend auf den mittels der einzelnen Drucksensoren zum Erfassungszeitpunkt erfassten Kräfte, die von der Druckerfassungseinrichtung wurden; und das Ausgeben des Abbild Kräfteverteilung des Fußes. Die Drucksensoren der Druckerfassungseinrichtung können dabei eine Dichte von mindestens 20, bevorzugt von mindestens 25 Drucksensoren pro 1 cm², ferner bevorzugt von mindestens 35 Drucksensoren pro 1 cm², und weiter bevorzugt mindestens 40 Drucksensoren pro 1 cm² aufweisen.

In dem computer-gestützten Verfahren kann in einer weiteren Ausführungsform der Erfindung die Verarbeitungseinrichtung die Messung der auf die Fläche der Druckerfassungseinrichtung wirkenden Kräfte des Fußes mittels eines Erfassungssignals veranlassen. Dazu kann die Verarbeitungseinrichtung beispielsweise unterschiedliche Erfassungssignale an die Druckerfassungseinrichtung übermitteln, um deren Funktion zu steuern, wie dies bereits beispielhaft dargelegt wurde.

In einer weiteren Ausführungsform können die von der Druckerfassungseinrichtung ermittelten Messwerte einzelne Sätze von Messwerten umfassen. Jeder Satz von Messwerten kann dabei die auf die Fläche der Druckerfassungseinrichtung wirkenden Kräfte des Fußes auf die Drucksensoren zu einem jeweiligen von mehreren periodischen Erfassungszeitpunkten repräsentieren. Die Verarbeitungseinheit kann basierend auf den Sätzen von Messwerten eine zeitliche Sequenz mehrerer zweidimensionaler Abbilder der Kräfteverteilung des Fußes auf der Druckerfassungseinrichtung erstellen.

Gemäß einer weiteren Ausführungsform des Computer-gestützten Verfahrens kann die Verarbeitungseinheit ausgebildet sein, aus den Abbildern der Kräfteverteilung des Fußes auf der Druckerfassungseinrichtung durch Mittelwertbildung der jeweiligen zu den unterschiedlichen Messzeitpunkten auf die jeweiligen Drucksensoren wirkenden Kräfte einen dynamischen Fußabdruck zu erzeugen und/oder auszugeben.

In einer Weiterbildung dieser Ausführungsform umfassen die Messwerte einzelne Sätze von Messwerten, wobei jeder Satz von Messwerten die auf die Fläche der Druckerfassungseinrichtung wirkenden Kräfte des Fußes auf die Drucksensoren zu einem jeweiligen von mehreren periodischen Erfassungszeitpunkten repräsentiert. Ferner ermittelt die Verarbeitungseinheit für jeden der Drucksensoren bevorzugt einen Mittelwert der durch den jeweiligen Drucksensor zu den unterschiedlichen Erfassungszeitpunkten erfassten Kräfte und erstellt basierend auf den Mittelwerten ein zweidimensionales dynamisches Abbild der Kräfteverteilung des Fußes auf der Druckerfassungseinrichtung.

In einer weiteren Ausführungsform des Computer-gestützten Verfahrens kann die Druckerfassungseinrichtung die auf jeden Drucksensor in einem Messintervall maximal wirkende Druckkraft zu ermitteln, die von dem Fuß auf die Fläche der Druckerfassungseinrichtung auf den jeweiligen Drucksensor ausgeübt wird. Die Verarbeitungseinheit erstellt, basierend auf den maximalen Druckwerten ein zweidimensionales statisches Abbild der Kräfteverteilung des Fußes auf der Druckerfassungseinrichtung im Messintervall.

Die erfassten Messwerte können die auf jeden Drucksensor in einem zweiten Messintervall maximal wirkende Druckkraft repräsentieren, die von dem Fußund/oder einem Eingabemittel, mit dem der Fuß umfahren wird, auf die Fläche der Druckerfassungseinrichtung auf den jeweiligen Drucksensor ausgeübt werden. Die Verarbeitungseinheit erstellt basierend auf den maximalen Druckwerten ein zweites zweidimensionales statisches Abbild der Kräfteverteilung des Fußes und des Eingabemittels auf der Druckerfassungseinrichtung im Messintervall.

In einer Weiterbildung der Ausführungsform ermittelt die Verarbeitungseinheit den mit dem Eingabemittel erzeugten Teil der im Erfassungsintervall auf die Fläche wirkenden Kräfte durch Bildung eines Differenzbildes aus dem zweiten zweidimensionalen Abbild und dem ersten zweidimensionalen Abbild der Kräfteverteilung des Fußes auf der Druckerfassungseinrichtung und erzeugt daraus ein Abbild des Umrisses des Fußes. Optional kann die Verarbeitungseinheit dabei Druckdifferenzen im Differenzbild, die kleiner einem vorgegeben oder konfigurierbaren Schwellenwert sind, bei der Ermittlung des Umrisses ignorieren.

In einer weiteren beispielhaften Ausführungsform ist die Verarbeitungseinheit eingerichtet, basierend auf dem ermittelten Umriss des Fußes die Fußmittelachse zu bestimmen.

Es ist gemäß einer weiteren Ausführungsform möglich, dass die Verarbeitungseinheit sowohl die Fußmittelachse für einen Umriss des Fußes, der auf einem Mittelwert-basierten, zweidimensionalen Abbild der Kräfteverteilung des Fußes ermittelt wurde, als auch die Fußmittelachse für einen Umriss des Fußes, der auf einem Einzelmessung-basierten zweidimensionalen Abbild der Kräfteverteilung des Fußes ermittelt wurde, errechnet.

Gemäß einer weiteren Ausführungsform des Computer-gestützten Verfahrens kann die Verarbeitungseinheit bewirken, dass der Umriss des Fußes und das zweidimensionale Abbild der Kräfteverteilung des Fußes auf einer Anzeigeeinheit ausgegeben wird.

Optional kann die Verarbeitungseinheit dabei bewirken, dass die unterschiedlichen Kräfte des zweidimensionalen Abbilds der Kräfteverteilung des Fußes entsprechend der jeweiligen Position der Drucksensoren in der vorgegebenen zweidimensionalen Anordnung in unterschiedlichen Farben und/oder Farbabstufungen dargestellt werden.

Die Verarbeitungseinheit bewirkt in einer weiteren Ausführungsform der Erfindung, dass der Umriss des Fußes als Umrisslinie in der Darstellung des zweidimensionalen Abbilds der Kräfteverteilung des Fußes gekennzeichnet wird. Es ist ebenfalls möglich, dass die Verarbeitungseinheit veranlasst, dass die Fußmittelachse in der Darstellung des zweidimensionalen Abbilds der Kräfteverteilung des Fußes gekennzeichnet wird.

### Kurze Beschreibung der Figuren

Verschiedene vorteilhafte Ausführungsformen der Erfindung werden nachfolgend in Bezug auf die beigefügten Zeichnungen näher erläutert. Es zeigen:
- **Figur 1A**: zeigt ein digitales Abbild der Druckverteilung eines Fußes auf einer Druckplatte gemäß einer Ausführungsform der Erfindung;
- **Figur 1B**: zeigt das in Fig. 1A dargestellte Abbild der Druckverteilung eines Fußes mit einem ermittelten Umriss des Fußes;
- **Figur 1C**: zeigt das in Fig. 1B dargestellte Abbild der Druckverteilung eines Fußes, mit Umriss des Fußes und mit einem ermittelten Umriss einer Schuheinlage bzw. einer Sohle;
- **Figur 2**: zeigt eine beispielhafte Ausführungsform eines Systems 100 zur Erfassung eines Fußabdrucks mittels einer Druckerfassungseinrichtung 102;
- **Figur 3**: zeigt exemplarisch eine Aufsicht in z-Richtung auf die Druckplatte 102 der Figur 2 gemäß einer Ausführungsform der Erfindung;
- **Figur 4**: zeigt ein Flussdiagramm eines Computer-gestützten Verfahrens gemäß einer Ausführungsform der Erfindung, das von einer Verarbeitungseinheit 110 ausgeführt wird.

### Detaillierte Beschreibung der Erfindung

Bevor nachfolgend verschiedenen Ausführungsformen der vorliegenden Erfindung anhand der Zeichnungen und Figuren näher erläutert werden, ist festzuhalten, dass identische, funktionsgleiche oder gleichwirkende Elemente bzw. Mittel in den unterschiedlichen Figuren mit den gleichen Bezugszeichen versehen sind. Entsprechend ist die in den unterschiedlichen Ausführungsformen dargestellte Beschreibung dieser Elemente untereinander austauschbar bzw. können diese aufeinander angewendet werden.

Ein Aspekt der vorliegenden Erfindung besteht darin, die Auflösung der Abbildung der Druckverteilung eines Fußes durch das Vorsehen einer besonders großen Anzahl von Drucksensoren pro Flächeneinheit in der elektronischen Druckplatte signifikant zu erhöhen. In den verschiedenen Ausführungsformen, die nachstehend beispielhaft erläutert werden, umfasst eine Druckplatte ca. 30.000 oder mehr einzelne kapazitive Drucksensoren auf einer Fläche von ca. 40 cm x 20 cm. Durch den Einsatz dieser Vielzahl von Sensoren, lassen sich die ortsabhängigen Druckverhältnisse auf die Oberfläche der Druckplatte, die von dem Fuß des Probanden/der Probandin ausgeübt werden, hochauflösend digital abbilden. Anhand des digitalen Abbilds der Druckverteilung des Fußes auf der Druckplatte kann die Druckverteilung exakt erfasst werden, so dass basierend auf den digital erfassten Daten der Druckverteilung des Fußes individuell angefertigte (orthopädische) Schuheinlagen mit großer Präzision gefertigt werden können. Die so erfassten Abdrücke lassen sich beispielsweise auf einer Anzeigevorrichtung (z.B. ein Bildschirm) darstellen. Die Darstellung der Druckverteilung kann beispielsweise in einem Blauabdruck Modus, einem Echtfarb-Modus oder in einer beliebigen Farbskala erfolgen, die die unterschiedlichen Druckwerten unterschiedlichen Farben zugeordnet. Es ist ebenfalls möglich, prozentuale Werte über die Lastverteilung des Fußes auf die vorgegebene Fläche der Druckplatte zu errechnen und darzustellen.

Die Druckerfassungseinrichtung kann in unterschiedlichen Modi betrieben werden. So können beispielsweise dynamische Aufnahmen der Druckverteilung aufgenommen werden. Dabei steht der Proband für ein vorbestimmtes oder vorgegebenes Erfassungsintervall, beispielsweise mindestens 1 Sekunde bis zu 240 Sekunden (oder auch mehr) auf der Druckerfassungseinrichtung (z.B. elektronische Druckplatte), wobei in dieser Zeit die Druckerfassungseinrichtung mehrere Messungen der Druckverteilung erfasst. Aus den so gewonnenen unterschiedlichen Messungen kann durch Mittelwertbildung ein statisches Abbild der Druckverteilung erzeugt werden.

Bei der Erfassung eines statischen Fußabdrucks werden die auf die jeweiligen Drucksensoren wirkende maximale Kraft während einer Messperiode erfasst. In einem anderen Betriebsmodus kann optional zusätzlich zur statischen Erfassung der Druckverteilung auch der Umriss des Fußes erfasst werden. Dazu verbleibt der Proband beispielsweise auf der Druckerfassungseinrichtung. Beispielsweise kann nach der Erfassung des statischen Fußabdrucks (oder davor) in einer weiteren Messperiode der Fuß des Probanden auf der Druckerfassungseinrichtung mittels eines Eingabemittels nachgezeichnet werden, indem dem Umriss des Fußes entlang Druck auf die Drucksensoren ausgeübt wird. Dieser durch die Eingabemittel ausgeübte Druck wird in einer weiteren Messung erfasst. Durch die Berechnung eines Differenzbilds zwischen der ersten und der zweiten Messung kann so der Fußumriss des Probanden/der Probandin ermittelt werden. Die Umrisserfassung des Fußes in digitaler Form ist eine Innovation, die heute noch nicht technisch umgesetzt ist. In einem weiteren Betriebsmodus lassen sich beim Laufen über die Druckerfassungseinrichtung beliebig viele dynamische Bilder zu generieren, auch hier kann das Einzelbild oder das durchschnitt Bild dargestellt werden. Es können alle Messdaten (statisch/dynamisch/Umriss) einzeln oder gemeinsam erfasst und gegenübergestellt werden. Der Fußabdruck wird in der Statik, sowie in der Dynamik mittels einer Linie an der Außentangente und Innentangente versehen. Aus diesen Tangenten kann anschließend die Fußmittelachse errechnet werden.

Wie eingangs bereits erwähnt ist ein Hauptproblem der heute gängigen elektronischen Druckplatten, die die digitale Erfassung von Fußabdrücken ermöglichen, dass diese nur eine unzureichende Ortsauflösung der Druckverhältnisse auf die vorgegebene Fläche ermöglichen. Aufgrund verschwommener Konturen lässt sich basierend auf solchen Systemen beispielsweise die Fußlänge oder gar ein Fußumriss nur schwer und sehr ungenau ermitteln. -

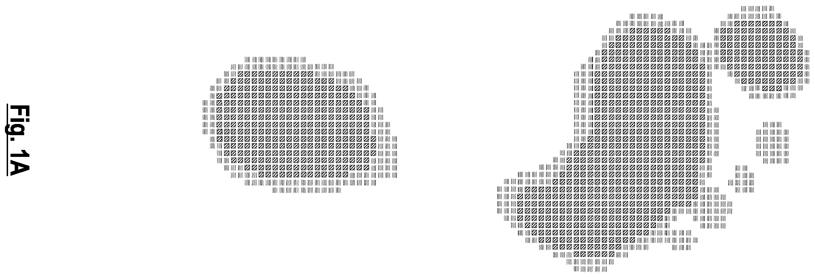

Diese Unzulänglichkeiten in den digitalen Abbildungen konventioneller Sensorbestückter Druckplatten lassen sich mit der Druckerfassungseinrichtung gemäß der vorliegenden Erfindung beheben. Durch die ausreichend gute Auflösung des Abbilds der Druckverteilung auf der Druckerfassungseinrichtung, die beispielsweise in Figur 1A dargestellt ist, können die erforderlichen Parameter zur Vermessung des Fußes des Probanden mit hoher Genauigkeit bestimmt werden.

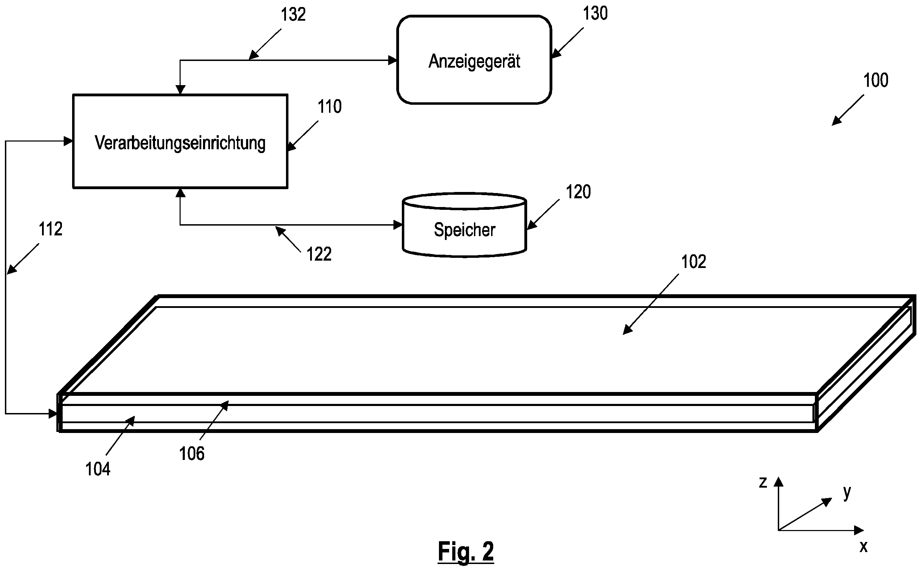

Figur 2 zeigt eine beispielhafte Ausführungsform eines Systems 100 zur Erfassung eines Fußabdrucks mittels einer Druckerfassungseinrichtung 102. Das System 100 umfasst eine Druckerfassungseinrichtung 102, im folgenden auch Druckplatte 102.Die Druckplatte 102 ist flächig (in einer x-y Ebene) ausgebildet. Die Druckplatte 102 kann eine Fläche von ca. 40 cm x 20 cm aufweisen. Die Dicke der Druckplatte 102 (z-Richtung) beträgt beispielsweise nur wenige Zentimeter (z.B. kleiner gleich 2 cm).

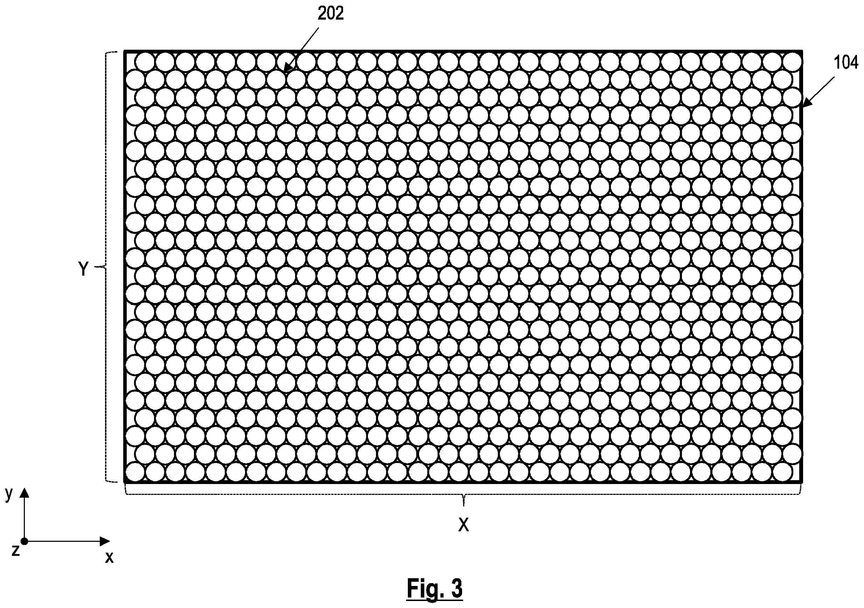

Die Größe der Fläche (x-y-Ebene) der Druckplatte 102 kann beliebig den äußeren Umständen angepasst werden. Sie sollte so groß sein, dass zumindest ein Fuß der Probanden auf der Oberfläche platziert werden kann und die Drucksensoren 104 der Druckplatte 102 die auf die Oberfläche der Druckplatte 102 in z-Richtung wirkenden Kräfte akkurat messen können. Zusätzlich kann die Druckplatte 102 beispielsweise auch weitere Indikatoren visueller Art (z.B. eine eigene Anzeigevorrichtung, beispielweise in Form eines LCD oder OLEDs basierten Displays) umfassen, um beispielsweise den aktuellen Betriebszustand/-Modus der Druckplatte 102 dem Benutzer kenntlich zu machen oder dem Benutzer Anweisungen zu geben, wie er die Druckplatte 102 benutzen soll. Es ist auch denkbar, dass die Fläche der Druckplatte 102 so gewählt ist, dass der Proband/die Probandin mit beiden Füßen auf der Druckplatte 102 stehen kann, sodass gleichzeitig oder aufeinanderfolgend der Fußabdruck und oder Umriss sowohl des linken als auch des rechten Fußes erfasst werden kann. Wie nachstehend noch mehr in Zusammenhang mit der Figur 3 erläutert werden wird, kann gemäß einer Ausführungsform der Erfindung das Seitenverhältnis der Druckplatte 102 im Bereich 1:1,5 und 1:2,5 liegen und bevorzugt 1:2 sein.

Die Druckplatte 102 umfasste ferner eine Vielzahl an Drucksensoren 202, die auch als Sensor-Array 104 bezeichnet werden können bzw. dieses bilden. Die Drucksensoren 202, die das Sensor-Array 104 bilden, sind ebenfalls flächig (in einer x-y Ebene) angeordnet. In der gezeigten Ausführungsform entspricht die Fläche des Sensor-Array 104 im Wesentlichen der Fläche der Druckplatte 102. In dem gezeigten Ausführungsbeispiel der Figur 2 ist das Sensor-Array 104 parallel zur Oberfläche der Druckplatte 102 angeordnet (d.h. in der x-y Ebene). Das Sensor-Array 104 kann beispielsweise in das die Oberseite bildende Material der Druckplatte 102 integriert sein, sodass sichergestellt wird, dass die Drucksensoren des Sensor-Array 104 nicht durch ein Auftreten, bzw. stehen des Probanden/der Probanden auf der Oberfläche der Druckplatte 102 beschädigt wird.

Alternativ kann das Sensor-Array 104 durch eine über dem Sensor-Array 104 angeordnete schützende Folie, Matte oder Platte 106 geschützt werden. Diese Folie, Matte oder Platte 106 sollte so ausgebildet sein, dass sie die auf sie einwirkenden Kräfte des Fußes, wenn der Proband/die Probandin über die Druckplatte 102 läuft oder auf ihr steht möglichst nicht in der Fläche verteilt, sodass die einzelnen Drucksensoren des Sensor-Array 104 die einwirkenden Kräfte möglichst ortsgenau erfassen können. Beispielsweise kann eine dünne Gummimatte 106 über dem Sensor-Array 104 angebracht sein, um die Drucksensoren zu schützen.

Die Druckplatte 102 umfasst auch die notwendige Elektronik und/oder Software (nicht gezeigt), um die einzelnen Drucksensoren 202 des Sensor-Array 104 anzusteuern bzw. auszulesen und um die erfassten Sensordaten zwischenzuspeichern und/oder and die Verarbeitungseinrichtung 110 zu übertragen. Die Druckplatte 102 reagiert auf ein Erfassungssignal, das von der Verarbeitungseinrichtung 110 über eine Schnittstelle 112 an die Druckplatte 102 übermittelt wird. Die Schnittstelle 112 kann dabei als drahtlose Schnittstelle (z.B. Bluetooth, WLAN, entsprechend einem Mobilfunkstandard, etc.) oder drahtgebundene Schnittstelle ausgebildet sein (z.B. USB). Je nach Art des Erfassungssignals, kann die Druckplatte 102 in verschiedenen Betriebs-Modi betrieben werden, bzw. in verschiedene Betriebszustände versetzt werden. Die Verarbeitungseinrichtung 110 kann so die Druckplatte 102 mittels der Erfassungssignale ansteuern, um beispielsweise einen statischen Fußabdruck, einen dynamischen Fußabdruck oder den Fußumriss des Probanden/der Probanden zu erfassen. Ferner kann die Verarbeitungseinrichtung 110 auch verschiedene Einstellungen an der Druckplatte 102 vornehmen, wie zum Beispiel die Erfassungsintervalle bzw. Länge der Messperioden einstellen.

Neben der Druckplatte 102 umfasst das System 100 auch die Verarbeitungseinrichtung 110. Es ist auch denkbar, dass die Verarbeitungseinrichtung 110 im Gehäuse der Druckplatte 102 integriert wird, so dass die Druckplatte 102 und die Verarbeitungseinrichtung 110 eine Einheit, z.B. ein Gerät bilden. Entsprechend würde in diesem Falle die Schnittstelle 112 eine interne Schnittstelle darstellen.

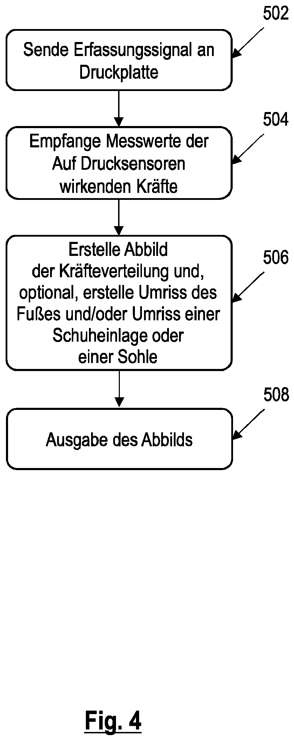

Die Verarbeitungseinrichtung 110 kann beispielsweise einen Prozessor umfassen, der ein Computer-implementiertes Verfahren ausführt (vgl. auch Figur 4), mittels dessen ein Fußabdruck unter Zuhilfenahme der Druckplatte 102 erfasst werden kann. Neben einem Prozessor kann die Verarbeitungseinrichtung 110 auch weitere (herkömmliche Komponenten) einer Computer-Vorrichtung, wie beispielsweise einen flüchtigen Speicher, ein nicht-flüchtigen Speicher, etc. aufweisen, die einen entsprechenden Programmcode und/oder eine Firmware speichern, die vom Prozessor ausgeführt wird. Die Verarbeitungseinrichtung 110 kann beispielsweise mittels eines Tablet-Computers, eines Smartphones, einem Laptop, oder stationären PC realisiert werden. Die Erfindung ist jedoch nicht auf diese Ausführungsformen beschränkt.

Die Verarbeitungseinrichtung 110 kann die erfassten Messdaten und/oder Abbildungen der Kräfteverteilung des Fußes auf der Druckplatte 102, die von der Druckplatte 102 erhalten werden auch extern speichern. Dazu ist exemplarisch ein Speicher 120 in der Figur 2 gezeigt. Dieser könnte beispielsweise ein Cloud-Speicher oder ein Netzwerkspeicher in einem lokalen Netzwerk sein. Die Verarbeitungseinrichtung 110 umfasst entsprechend auch eine Schnittstelle 122, um die Daten an den Speicher 120 zu übertragen und gegebenenfalls auch Daten aus dem Speicher 120 zu empfangen. Ferner kann die Verarbeitungseinrichtung 110 auch an ein Anzeigegerät 130 gekoppelt sein, auf dem die Messdaten und/oder Abbildungen der Kräfteverteilung des Fußes auf der Druckplatte 102 dem Benutzer zur Anzeige gebracht werden können. Bei dem Anzeigegerät 130 kann es sich beispielsweise um einen Bildschirm handeln. Das Anzeigegerät 130 kann optional auch in der Verarbeitungseinrichtung 110 integriert sein, beispielsweise wenn es sich bei der Verarbeitungseinrichtung 110 um einen Tablet-Computer, ein Smartphone oder einen Laptop handelt.

Die Verarbeitungseinrichtung kann mittels des Anzeigegeräts 130 dem Benutzer des Systems 100 auch eine Benutzeroberfläche zur Verfügung stellen, mit der der Benutzer mit dem System 100 interagieren kann. Mittels dieser Benutzerschnittstelle kann der Benutzer beispielsweise den Betriebsmodus der Druckplatte 102 einstellen, unterschiedliche Erfassungsvorgänge starten und Einstellungen an der Druckplatte 102 vornehmen. Ferner kann die Benutzerschnittstelle dazu dienen, verschiedene Auswertungen der erfassten Messdaten zu erzeugen und diese auf dem Anzeigegerät 130 anzuzeigen. Beispielsweise kann die Benutzerschnittstelle dem Benutzer ermöglichen, die Kräfteverteilung auf unterschiedliche Arten und Weisen auf dem Anzeigegerät darzustellen, den Umriss des Fußes zu kennzeichnen, die äußeren und inneren Tangenten des Fußumrisses zu kennzeichnen, die Fußmittelachse zu kennzeichnen und/oder die Fußlänge anzuzeigen.

Die Verarbeitungseinrichtung kann ferner über weitere Schnittstellen umfassen, die die erfassten Messdaten und Analysedaten direkt und in digitaler Form in eine Produktionsanlage zur Herstellung orthopädische Schuheinlagen eingespeist werden können.

Figur 3 zeigt exemplarisch eine Aufsicht in z-Richtung auf die Druckplatte 102 der Figur 2. Die Druckplatte 102 umfasst über eine Vielzahl von Drucksensoren 202, die das in Figur 2 gezeigte Sensor-Array 104 bilden. Die einzelnen Drucksensoren 202 sind in der Figur 3 exemplarisch als Kreise dargestellt. Die Drucksensoren 202 sind dabei flächig in einer x-y Ebene angeordnet. Die Drucksensoren 202 können beispielsweise kapazitive Drucksensoren sein, die die Änderung einer Kapazität in Reaktion auf eine in z-Richtung auf die Druckplatte 102 bzw. auf den jeweiligen Drucksensor 202 einwirkende Kraft erfassen. Diese Änderung der Kapazität kann proportional zu der auf den jeweiligen Drucksensor 202 einwirkenden Druckkraft sein. Das Seitenverhältnis X:Y des Sensor-Arrays 104 (d.h. der flächigen Anordnung der Drucksensoren 202) kann beispielsweise im Bereich 1:1,5 und 1:2,5 liegen, und kann bevorzugt 1:2 sein. In dem in Figur 3 gezeigten Ausführungsbeispiel, entspricht die Fläche des Sensor-Array 104 im Wesentlichen der Fläche der Druckplatte 102. Jedoch kann die Abmessung der Druckplatte 102 natürlich auch größer als die Fläche des Sensor-Arrays 104 sein.

Die Anzahl der Drucksensoren 202 im Sensor-Array 104 ist so gewählt, dass die Dichte der Drucksensoren in Bezug auf die Fläche (in x-y Ebene) 25 Drucksensoren 202 oder mehr pro 1 cm², bevorzugt mindestens 35 Drucksensoren 202 oder mehr pro 1 cm², und weiter bevorzugt mindestens 40 Drucksensoren 202 oder mehr pro 1 cm² beträgt. Diese Dichte ermöglicht eine ausreichend hohe Genauigkeit und Ortsauflösung bei der Erfassung eines Fußabdrucks, d. h der auf die Fläche der Druckplatte 102 wirkenden Kräfte des Fußes, die mittels des Sensor-Array 104 erfasst werden (siehe Figur 1A).

Figur 4 zeigt ein exemplarisches Flussdiagramm eines Computer-gestützten Verfahrens, das beispielsweise von der Verarbeitungseinheit 110 ausgeführt wird. Wie bereits erwähnt, kann die Verarbeitungseinheit 110 die Druckplatte 102 auf unterschiedliche Art und Weise ansteuern, um so die Erfassung eines dynamischen Fußabdrucks, eines statischen Fußabdrucks oder die Erfassung des Fußumrisses zu initiieren. Dazu verwendet die Verarbeitungseinheit 110 unterschiedliche Erfassungssignale, die die Verarbeitungseinrichtung 110 and die Druckplatte 102 sendet 502. Nachdem die Druckplatte 102 die entsprechende Messung ausgeführt hat, empfängt 504 die Verarbeitungseinheit 110 die entsprechenden Messwerte der Drucksensoren 202 von der Druckplatte 102. Die Messwerte repräsentieren die auf die jeweiligen Drucksensoren der Druckerfassungseinrichtung wirkenden Kräfte eines Fußes zu einem gegebenen Erfassungszeitpunkt. Je nach Betriebsmodus der Druckplatte 102 umfassen die Messwerte mehrere Sätze von Messwerten, wobei jeder Satz von Messwerten die auf die Fläche der Druckplatte 102 wirkenden Kräfte des Fußes auf die Drucksensoren 202 zu einem jeweiligen von mehreren periodischen Erfassungszeitpunkten in einer Messperiode repräsentieren kann.

Die Verarbeitungseinrichtung 110 erstellt 506 ein Abbild (oder mehrere Abbilder) der Kräfteverteilung basierend auf den von der Druckplatte 102 empfangenen Messwerten. Dieses Erstellen 506 eines zweidimensionalen Abbilds kann dabei mehrere Rechenoperationen umfassen.

Beispielsweise kann bei der Ermittlung eines dynamischen Fußabdrucks die Druckplatte 102 in Reaktion auf ein entsprechendes Erfassungssignal, das von der Verarbeitungseinrichtung 110 an die Druckplatte gesendet 502 wird, mehrere Sätze von Messwerten der Drucksensoren 202 an die Verarbeitungseinheit 110 übertragen. Die einzelnen Sätze der Messwerte entsprechen einzelnen Messungen der jeweiligen Normalkraft, mit der die einzelnen Drucksensoren 202 während eines Auftretens und Abrollen des Fußes auf der Druckplatte 102 beaufschlagt werden. Der Vorgang des Auftretens und Abrollen des Fußes beträgt in der Regel ca. 1 bis 2 Sekunden. Entsprechend erfasst die Druckplatte 102 in einer vorgegebenen oder (mittels des Erfassungssignals oder anderweitig durch die Verarbeitungseinrichtung 110) konfigurierbaren Messperiode Pd (z.B. 2 Sekunden, 3 Sekunden, 4 Sekunden, 5 Sekunden, etc. oder auch länger) in kurzen zeitlichen Intervallen T (periodisch, z.B. jede 10 Millisekunden) die momentan auf die jeweiligen Drucksensoren 202 einwirkenden Kräfte und übermittelt diese als einzelne Sätze von Messwerten der Drucksensoren 202 des Sensor-Array 104 an die Verarbeitungseinheit 110. Die zeitlichen Intervalle T zwischen den einzelnen Messungen können beispielsweise in einem Bereich von 5 ms bis 30 ms, und bevozugt 10 ms bis 20 ms, liegen. In einem Ausführungsbeispiel beträgt das Intervall T 5ms, 10 ms, 15 ms oder 20 ms.

Entsprechend erzeugt die Druckplatte 102 in der Messperiode Pd gegebenenfalls mehr als 100 Sätze von Messwerten, die an die Verarbeitungseinrichtung 110 übermittelt werden. Die Verarbeitungseinrichtung 110 kann jeden dieser Sätze von Messwerten (oder einen Teil davon) in eine zweidimensionale Abbildung umwandeln, in der die jeweiligen Druckwerte in Abhängigkeit von der jeweiligen Position des Drucksensors in der x-y Ebene des Sensor-Array 104 beispielsweise mit unterschiedlichen Farben oder Graustufen gekennzeichnet werden. Jedes dieser Abbilder entspricht somit der Druckverteilung auf der Druckplatte 102 zum jeweiligen Erfassungszeitpunkt des Satzes von Messwerten durch die Druckplatte 102. Die Vorrichtungseinheit 110 kann dabei beispielsweise angepasst sein, mithilfe der Anzeigevorrichtung 130 die einzelnen Abbilder der Druckverteilung auf der Druckplatte 102 in einer zeitlichen Sequenz dem Benutzer darzustellen, sodass der Benutzer die zeitliche Änderung der Druckverhältnisse optisch wahrnehmen und analysieren/auswerten kann. Um einen dynamischen Fußabdruck zu generieren bildet die Verarbeitungseinheit 110 für jeden der Sensoren 202 einen Mittelwert aus den erfassten Druckwerten des jeweiligen Drucksensors 202 in den unterschiedlichen Sätzen von Messwerten. Daraus ergibt sich ein Durchschnitts-Abbild der Mittelwerte des Fußabdrucks (dynamischen Fußabdruck), dass von der Verarbeitungseinrichtung 110 beispielsweise auf dem Anzeigegerät 130 zur Anzeige gebracht werden kann.

Zur Ermittlung eines statischen Fußabdrucks veranlasst die Verarbeitungseinrichtung 110 die Druckplatte 102 mithilfe eines entsprechenden Erfassungssignals dazu, die auf jeden Drucksensoren 202 in einem Messintervall Ps maximal wirkende Druckkraft auf den jeweiligen Drucksensoren 202 zu ermitteln, die von dem Fuß auf die Fläche der Druckplatte 102 bzw. den jeweiligem Drucksensor 202 ausgeübt wird. Das Messintervall Ps kann dabei ebenfalls mittels des Erfassungssignals oder anderweitig durch die Verarbeitungseinrichtung 110 konfiguriert oder vorgegeben werden. Das Messintervall Ps kann beispielsweise zwischen 1 Sekunde und 240 Sekunden liegen. Typischerweise liegt das Messintervall Ps für einen statischen Fußabdruck im Bereich von ca. 10 Sekunden, bevorzugt 5 Sekunden, 10 Sekunden oder 15 Sekunden. Nach Ablauf des Messeintervalls Ps übermittelt die Druckplatte 102 einen Satz an Druckwerten für die Drucksensoren 202 des Sensor-Array 104 an die Verarbeitungseinrichtung 110. Die Verarbeitungseinrichtung 110 wandelt diesen Satz von Messwerten in eine zweidimensionale Abbildung um, in der die jeweiligen Druckwerte in Abhängigkeit von der jeweiligen Position des Drucksensors in der x-y Ebene des Sensor-Array 104 beispielsweise mit unterschiedlichen Farben gekennzeichnet werden. Die Vorrichtungseinheit 110 kann dabei beispielsweise angepasst sein, mithilfe der Anzeigevorrichtung 130 das so erzeugte Abbild der Druckverteilung auf der Druckplatte 102 (statischen Fußabdruck) anzuzeigen.

Sofern mithilfe der Druckplatte 102 der Umriss des Fußes auf der Druckplatte 102 nachgezeichnet werden und erfasst werden soll, veranlasst die Verarbeitungseinrichtung 110 die Druckplatte 102 in einen Betriebsmodus einzutreten, der dem der Erfassung des statischen Fußabdrucks im Wesentlichen entspricht. Das Messintervall Ps, im Zusammenhang mit der Erfassung des Fußumrisses als Messintervall Pu bezeichnet, kann dabei etwas länger gewählt werden, damit der Benutzer ausreichend Zeit hat, den Umriss des Fußes auf der Druckplatte 102 zu kennzeichnen. Auch hier kann das Messintervall Pq prinzipiell im Bereich zwischen 1 Sekunde und 240 Sekunden liegen. Vorteilhafterweise liegt das Messintervall Pu zur Erfassung des Fußumrisses im Bereich von 20 Sekunden oder mehr, damit der Benutzer ausreichend Zeit hat, den Fuß des Probanden/der Probandin zu umfahren.

Zur Erfassung des Fußumrisses kann der Benutzer den Fuß des Probanden/der Probandin mit einer beliebigen Eingabemittel umfahren, mittels dessen ein in z-Richtung wirkender Druck auf die Drucksensoren 202 ausgeübt werden kann. Dies kann beispielsweise ein (passiver) Stift sein, ein Kunststoffstab, oder dergleichen. Prinzipiell wäre es auch möglich, mithilfe eines Fingers den Fußumriss zu unterzeichnen, was jedoch aufgrund der niedrigen Auflösung in der Regel unvorteilhaft erscheint.

Wie auch bei der Erfassung eines statischen Fußabdrucks, erfasst die Druckplatte 102 die maximale Druckkraft, die auf jeden der Drucksensoren während des Messintervall einwirkt und sendet einen Satz von Messwerten an die Verarbeitungseinrichtung 110. Die Verarbeitungseinrichtung 110 wandelt diesen Satz von Messwerten in ein, in diesem Falle zweidimensionales, Abbild um. Um den Fußumrisses aus den Messwerten zu extrahieren, kann zusätzlich ein (weiteres) statisches Abbild eines Fußabdrucks auf die Druckplatte 102 in der beschriebenen Art und Weise erfasst werden. Die Verarbeitungseinrichtung 110 bildet aus diesem weiteren statischen Abbild und dem zweidimensionalen Abbild, das den Fußumriss enthält, ein Differenzbild, sodass lediglich der Fußumriss im Differenzbild verbleibt. Da während den unterschiedlichen Aufnahmen des statischen Fußabdrucks Druckschwankungen auftreten können, kann die Verarbeitungseinrichtung 110 eingerichtet sein, Druckdifferenzen im Differenzbild, die kleiner einem vorgegebenen oder konfigurierten Schwellenwert sind zu ignorieren bzw. die entsprechenden Differenzwerte kleiner dem Schwellenwert aus dem Differenzbild zu entfernen, sodass lediglich der Fußumrisses im Differenzbild verbleibt. Der so ermittelte Fußumrisses, bzw. sein Abbild, kann von der Verarbeitungseinrichtung 110 ebenfalls auf dem Anzeigegerät 130 zur Anzeige gebracht werden.

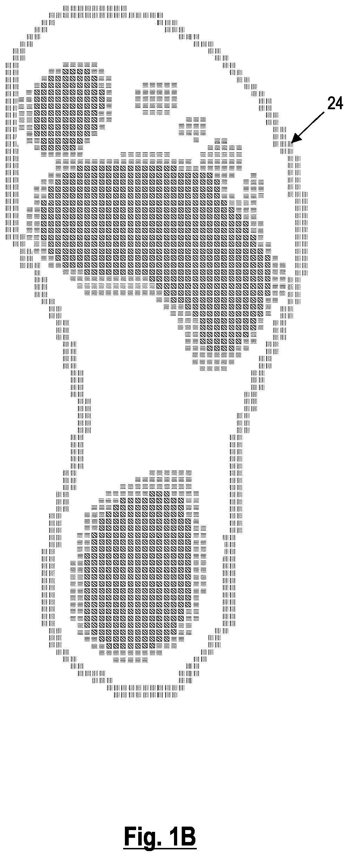

Fig. 1A zeigt ein digitales Abbild der Druckverteilung eines Fußes auf einer Druckplatte gemäß einer Ausführungsform der Erfindung, wobei aus Fig. 1A sehr gut die hohe Auflösung und die exakte Darstellung des Fußabdrucks ersichtlich ist.Fig. 1B zeigt das in Fig. 1A dargestellte Abbild der Druckverteilung eines Fußes mit einem ermittelten Umriss 24 des Fußes. Fig. 1B zeigt, in dieser Ausführungsform, sowohl die Druckverteilung des Fußes als auch den Umriss 24. Selbstverständlich ist es alternativ auch möglich, wie oben beschrieben, mittels Ermittlung eines Differenzbildes nur den Umriss 24 des Fußes darzustellen.

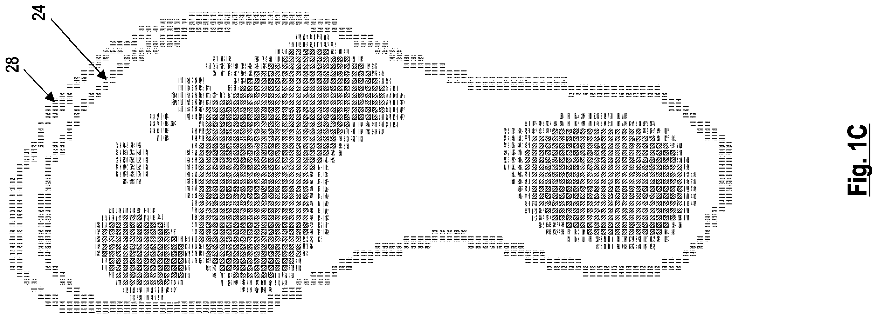

Fig. 1C schließlich zeigt die in Fig. 1B dargestellte Abbildung, bei der zusätzlich ein ermittelter Umriss 28 einer Schuheinlage bzw. einer Sohle dargestellt ist. Dieser Umriss 28 einer Schuheinlage bzw. einer Sohle kann beispielsweise durch automatische Vergrößerung des Umrisses, beispielsweise um 0,5 cm bis 1,5 cm oder um einen anderen festgelegten Wert, der beispielsweise auch in Abhängigkeit von den absolut gemessenen Druckwerten der einzelnen Drucksensoren und/oder einem entsprechenden Mittelwert und damit vom Gewicht des Nutzers abhängt, sodass auch eine bevorzugte Sohlengröße bestimmt werden kann.

Wie in Fig. 1C ersichtlich, wurde bei dieser Ausführungsform der Umriss 28 einer Schuheinlage bzw. einer Sohle nur im vorderen Bereich, insbesondere im Bereich der Zehen, gegenüber dem Umriss 24 des Fußes vergrößert.

Wie auch in Zusammenhang mit Fig. 1B beschrieben ist es alternativ auch möglich, lediglich den Umriss 28 einer Schuheinlage bzw. einer Sohle darzustellen, oder alternativ den Umriss 28 einer Schuheinlage bzw. einer Sohle und den Umriss 24 des Fußes darzustellen.

Optional kann die Verarbeitungseinrichtung 110 ferner eingerichtet sein, basierend auf dem Differenzbild, das den Fußumrisses darstellt, oder basierend auf dem Abbild eines statischen oder dynamischen Fußabdrucks die Fußmittelachse des Fußes zu bestimmen. Auch die Fußmittelachse kann entsprechend in einem der Abbilder gekennzeichnet werden und dem Benutzer mithilfe der Anzeigevorrichtung 130 zur Anzeige gebracht werden. Die Ermittlung der Fußmittelachse kann auch die Ermittlung der Außentangente und Innentangente des Fußes beinhalten, die zur Ermittlung der Fußmittelachse benötigt wird. Auch diese Tangenten können optional mithilfe der Anzeigevorrichtung 130, beispielsweise zusammen mit dem Umriss des Fußes und/oder einem dynamischen/statischen Abbild des Fußabdrucks zur Anzeige gebracht werden. Bei der Anzeige des dynamischen und/oder statischen Fußabdrucks auf der Anzeigevorrichtung 130 können die unterschiedlichen Kräfte des zweidimensionalen Abbilds der Kräfteverteilung des Fußes entsprechend der jeweiligen Position der Drucksensoren 2020 in der vorgegebenen zweidimensionalen Anordnung des Sensor-Array 104 in unterschiedlichen Farben und/oder Farbabstufungen dargestellt werden, wobei die einzelnen Farbwerte die unterschiedlichen Druckwerte kennzeichnen. Ferner kann gemäß einer weiteren Ausführungsform die Verarbeitungseinrichtung 110 basierend auf dem mittels Differenzbild-Bildung ermittelten Fußabdruck, einem dynamischen Fußabdruck und/oder einem statischen Fußabdruck die Fußlänge ermitteln und (ebenfalls) zur Anzeige bringen.

Wie bereits eingangs erwähnt, kann die Verarbeitungseinrichtung 110 auch anstelle der Anzeige der jeweiligen Abbilder des Umrisses und der dynamischen/statischen Fußabdrücke diese auch zur weiteren Verwendung in der Verarbeitungseinrichtung 110 selbst oder extern im Speicher 120 speichern. Einer oder mehrere der von Verarbeitungseinrichtung 110 ermittelten Parameter, wie die Fußmittelachse, statische und/oder dynamische Druckverteilung des Fußes, und die Fußlänge, können dazu verwendet werden, für den Probanden/die Probandin eine individuell angepasste (orthopädische) Schuheinlage zu fertigen. Dazu können die ermittelten Parameter, Fußumrisse, und dynamische/statische Fußabdrücke des Probanden/der Probandin beispielsweise an eine Orthopädiewerkstatt elektronisch übermittelt werden.

In weiteren Ausführungsformen der Erfindung, wird ein Teil der Funktionalität der Verarbeitungseinrichtung 110 in die Druckplatte 102 selbst integriert. Beispielsweise kann so die Druckplatte 102 die statische und/oder dynamischen Abbilder des Fußabdrucks selbst aus den Messwerten erstellen und an die Verarbeitungseinrichtung 110 übermitteln. Ebenso ist es denkbar, dass die Druckplatte 102 anstelle der Verarbeitungseinheit 110 den Fußumriss in der beschriebenen Art und Weise ermittelt und ein Abbild des Fußumrisses an die Verarbeitungseinrichtung 110 übermitteln. Weiter kann die Druckplatte 102 auch die Tangenten und Fußmittelachse ermitteln und an die Verarbeitungseinrichtung 110, beispielsweise, in einem der Abbilder für den Fußumriss, den statischen Fußabdruck, den dynamischen Fußabdruck oder als separates Abbild (das gegebenenfalls mit einem der anderen Abbilder überlagert werden kann) übermitteln.

## Patentansprüche

1. Vorrichtung aufweisend:
eine flächige Druckerfassungseinrichtung, die eine Vielzahl von Drucksensoren in einer vorgegebenen zweidimensionalen Anordnung beinhaltet, wobei die Druckerfassungseinrichtung eingerichtet ist, in Reaktion auf ein Erfassungssignal, die auf eine Fläche der Druckerfassungseinrichtung wirkenden Kräfte eines Fußes mittels der Drucksensoren zu einem Erfassungszeitpunkt zu messen;
eine Verarbeitungseinheit, die eingerichtet ist, die Messung der auf die Fläche der Druckerfassungseinrichtung wirkenden Kräfte des Fußes mittels des Erfassungssignal zu veranlassen und basierend auf den mittels der einzelnen Drucksensoren zum Erfassungszeitpunkt erfassten Kräften ein Abbild der Kräfteverteilung des Fußes auf der Druckerfassungseinrichtung zu erstellen,
wobei die vorgegebene zweidimensionale Anordnung eine Dichte von mindestens 20 Drucksensoren pro 1 cm², bevorzugt von mindestens 25 oder von mindestens 35 Drucksensoren pro 1 cm², und weiter bevorzugt mindestens 40 Drucksensoren pro 1 cm² aufweist.

2. Vorrichtung nach Anspruch 1, wobei:
die Drucksensoren in der Druckerfassungseinrichtung in einer im Wesentlichen rechteckigen Fläche mit einem Seitenverhältnis im Bereich 1:1,5 und 1:2,5, bevorzugt 1:2 angeordnet sind, und/oder
wobei die Drucksensoren in der Druckerfassungseinrichtung in einer im Wesentlichen rechteckigen Fläche von ca. 40cm x ca. 20 cm angeordnet sind.

3. Vorrichtung nach einem der Ansprüche 1 oder 2, wobei:
die Druckerfassungseinrichtung eingerichtet ist, in Reaktion auf ein erstes Erfassungssignal die auf die Fläche der Druckerfassungseinrichtung wirkenden Kräfte des Fußes mittels der Drucksensoren kontinuierlich in Intervallen zu unterschiedlichen Erfassungszeitpunkten zu ermitteln; und
die Verarbeitungseinheit eingerichtet ist, basierend auf den mittels der einzelnen Drucksensoren zu den unterschiedlichen Erfassungszeitpunkten erfassten Kräften eine zeitliche Sequenz mehrerer zwei- oder dreidimensionaler Abbilder der Kräfteverteilung des Fußes auf der Druckerfassungseinrichtung zu erstellen,
wobei bevorzugt die Verarbeitungseinheit ferner eingerichtet ist aus den Abbildern der Kräfteverteilung des Fußes auf der Druckerfassungseinrichtung durch Mittelwertbildung der jeweiligen zu den unterschiedlichen Messzeitpunkten auf die jeweiligen Drucksensoren wirkenden Kräfte einen dynamischen Fußabdruck zu erzeugen.

4. Vorrichtung nach einem der Ansprüche 1 bis 3, wobei:
die Druckerfassungseinrichtung eingerichtet ist, in Reaktion auf ein zweites Erfassungssignal die auf die Fläche der Druckerfassungseinrichtung wirkenden Kräfte des Fußes mittels der Drucksensoren kontinuierlich in Intervallen zu unterschiedlichen Erfassungszeitpunkten zu ermitteln; und
die Verarbeitungseinheit eingerichtet ist, für jeden der Drucksensoren einen Mittelwert der durch den jeweiligen Drucksensor zu den unterschiedlichen Erfassungszeitpunkten erfassten Kräfte zu ermitteln und basierend auf den Mittelwerten ein zwei- oder dreidimensionales dynamisches Abbild der Kräfteverteilung des Fußes auf der Druckerfassungseinrichtung zu erstellen.

5. Vorrichtung nach einem der Ansprüche 1 bis 4, wobei:
die Druckerfassungseinrichtung eingerichtet ist, in Reaktion auf ein drittes Erfassungssignal die auf jeden Drucksensor in einem Messintervall maximal wirkende Druckkraft zu ermitteln, die von dem Fuß auf die Fläche der Druckerfassungseinrichtung auf den jeweiligen Drucksensor ausgeübt wird; und
die Verarbeitungseinheit eingerichtet ist, basierend auf den maximalen Druckwerten ein statisches Abbild der Kräfteverteilung des Fußes auf der Druckerfassungseinrichtung im Messintervall zu erstellen.

6. Vorrichtung nach einem der Ansprüche 1 bis 5, wobei die Vorrichtung ferner ein Eingabemittel, insbesondere einen elektronischen, aktiven oder passiven, Stift umfasst, der so ausgebildet ist, dass mittels diesem der Fuß umfahren werden kann, und wobei bevorzugt mittels des Eingabemittels ein Druck auf die Fläche der Druckerfassungseinrichtung ausgeübt wird, der von entsprechenden Drucksensoren der Druckerfassungseinrichtung erfasst wird.

7. Vorrichtung nach einem der Ansprüche 1 bis 6, wobei:
die Druckerfassungseinrichtung eingerichtet ist, in Reaktion auf ein viertes Erfassungssignal die auf jeden Drucksensor in einem zweiten Messintervall maximal wirkende Druckkraft zu ermitteln, die von dem einem Eingabemittel , mit dem der Fuß umfahren wird, auf die Fläche der Druckerfassungseinrichtung auf den jeweiligen Drucksensor ausgeübt wird; und
die Verarbeitungseinheit eingerichtet ist, basierend auf den maximalen Druckwerten ein zweites statisches Abbild der Kräfteverteilung des Fußes und/ oder des Eingabemittel auf der Druckerfassungseinrichtung im Messintervall zu erstellen.

8. Vorrichtung nach Anspruch 7, wobei die Verarbeitungseinheit eingerichtet ist, den mit dem Eingabemittel erzeugten Teil der im Erfassungsintervall auf die Fläche wirkenden Kräfte durch Bildung eines Differenzbildes aus dem zweiten Abbild und dem ersten Abbild der Kräfteverteilung des Fußes auf der Druckerfassungseinrichtung zu ermitteln und daraus ein Abbild des Umrisses des Fußes zu erzeugen.

9. Vorrichtung nach Anspruch 8, wobei:
die Verarbeitungseinheit ferner eingerichtet ist, Druckdifferenzen im Differenzbild, die kleiner einem vorgegeben oder konfigurierbaren Schwellenwert sind, bei der Ermittlung des Umrisses zu ignorieren.

10. Vorrichtung nach Anspruch 8 oder 9, wobei:
die Verarbeitungseinheit ferner eingerichtet ist, basierend auf dem ermittelten Umriss des Fußes die Fußmittelachse zu bestimmen
wobei bevorzugt die Verarbeitungseinheit eingerichtet ist, sowohl die Fußmittelachse für einen Umriss des Fußes, der auf einem Mittelwert-basierten, Abbild der Kräfteverteilung des Fußes ermittelt wurde, als auch die Fußmittelachse für einen Umriss des Fußes, der auf einem Einzelmessung-basierten Abbild der Kräfteverteilung des Fußes ermittelt wurde, errechnet.

11. Vorrichtung nach einem der Ansprüche 1 bis 10, ferner umfassend:
eine Anzeigeeinheit, die eingerichtet ist, den Umriss des Fußes und das Abbild der Kräfteverteilung des Fußes anzuzeigen.

12. Vorrichtung nach einem der Ansprüche 1 bis 11, wobei:
die Verarbeitungseinheit eingerichtet ist, die unterschiedlichen Kräfte des Abbilds der Kräfteverteilung des Fußes entsprechend der jeweiligen Position der Drucksensoren in der vorgegebenen zweidimensionalen Anordnung in unterschiedlichen Farben und/oder Farbabstufungen darzustellen.

13. Vorrichtung nach einem der Ansprüche 1 bis 12, wobei:
die Verarbeitungseinheit eingerichtet ist, die den Umriss des Fußes als Umrisslinie in der Darstellung des Abbilds der Kräfteverteilung des Fußes zu kennzeichnen.

14. Vorrichtung nach einem der Ansprüche 1 bis 13, wobei:
die Verarbeitungseinheit eingerichtet ist, die Fußmittelachse in der Darstellung des Abbilds der Kräfteverteilung des Fußes zu kennzeichnen.

15. Computer-gestütztes Verfahren zur Ermittlung eines Fußabdrucks, wobei eine Verarbeitungseinheit, die bevorzugt eine Vorrichtung nach einem der vorhergehenden Ansprüche umfasst oder an eine solche Vorrichtung anschließbar ist, folgende Schritte ausführt:
Empfangen von Messwerten von einer Druckerfassungseinrichtung, wobei die Messwerte die auf die jeweiligen Drucksensoren der Druckerfassungseinrichtung wirkenden Kräfte eines Fußes zu einem Erfassungszeitpunkt repräsentieren;
Erstellen eines Abbilds der Kräfteverteilung des Fußes auf der Druckerfassungseinrichtung basierend auf den mittels der einzelnen Drucksensoren zum Erfassungszeitpunkt erfassten Kräfte, die von der Druckerfassungseinrichtung wurden; und
Ausgeben des Abbilds Kräfteverteilung des Fußes;
wobei die flächige Anordnung der Drucksensoren der Druckerfassungseinrichtung eine Dichte von mindestens 25 Drucksensoren pro 1 cm², bevorzugt von mindestens 35 Drucksensoren pro 1 cm², und weiter bevorzugt mindestens 40 Drucksensoren pro 1 cm² aufweist.
